(19) Europäisches Patentamt
European Patent Office
Office européen des brevets

(11) **EP 4 538 708 A1**

(12) **EUROPEAN PATENT APPLICATION**
published in accordance with Art. 153(4) EPC

(43) Date of publication:
**16.04.2025 Bulletin 2025/16**

(51) International Patent Classification (IPC):
**G01N 33/68** (2006.01)  **G16B 40/00** (2019.01)

(21) Application number: **23819074.8**

(52) Cooperative Patent Classification (CPC):
**G01N 33/68; G16B 40/00**

(22) Date of filing: **05.06.2023**

(86) International application number:
**PCT/CN2023/098376**

(87) International publication number:
**WO 2023/236909 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.06.2022 CN 202210638301**

(71) Applicant: **Southern University of Science and Technology**
**Shenzhen, Guangdong 518000 (CN)**

(72) Inventors:
• **JI, Hongchao**
  **Shenzhen, Guangdong 518000 (CN)**
• **TAN, Soonheng**
  **Shenzhen, Guangdong 518000 (CN)**

(74) Representative: **Ehrner & Delmar Patentbyrå AB**
**Götgatan 78**
**118 30 Stockholm (SE)**

(54) **METHOD FOR IMPROVING EXPERIMENTAL FLUX OF INTERACTION BETWEEN COMPOUNDS AND PROTEINS**

(57) The present application provides a method for improving a throughput of compound-protein interaction experiments. In the method of this application, multiple compounds to be tested are composed into multiple mixture systems according to a certain mixing rule, and corresponding relationships between abilities of the compounds to be tested to interact with the protein target and the mixture systems are established, and then the target protein corresponding to the compound to be tested is analyzed in a high-throughput manner. The analysis method of the present application can increase a detection throughput of the existing compound to be tested-target protein experiments by more than 10 times, while saving more than 90% of the experimental cost and time, significantly reducing the cost of manpower, time and experimental consumables, which has significant economic significance.

Matrix A is optimized and converted into Matrix S, to minimize the equation of: $Sum(S*S^T-I) + Sum(RS-Mean(RS))^2$

Matrix **A**  Matrix **S**  Correlation Matrix

Optimized Matrix **S**  Optimized Correlation Matrix

FIG. 1

## Description

This application claims the benefits of Chinese Patent Application No. 202210638301.1 filed with the China National Intellectual Property Administration on June 7, 2022, entitled "Method for Improving Throughput of Compound-Protein Interaction Experiments", the entire content of which is incorporated herein by reference.

## TECHNICAL FIELD

The present application relates to the field of computer-aided drug design technology, and in particular, to a method for improving a throughput of compound-protein interaction experiments.

## BACKGROUND

Compound molecules often regulate cellular processes through physical interactions with proteins in organisms, resulting in toxic and therapeutic effects. Identification of binding targets of compound molecules is usually solved by affinity-based or activity-based proteomic approaches (e.g., ABPP, PAL, KinoBeads), these approaches require chemical derivatization of compound molecules first, and the procedures are relatively complex. Non-derivatization mass spectrometry (MS) methods such as SPROX, TPP, DARTS, LiP-MS and CPP can be expanded to more compounds, but still require longer sample preparation and instrument measurement time.

## SUMMARY

The present application provides a method for improving the throughput of compound-protein interaction experiments to solve the technical problems of a high time-and-economic cost and a low detection throughput in the existing measurement experiment on interaction between a compound and a target protein.

To achieve the above objective of the present application, a method for improving a throughput of compound-protein interaction experiments is provided. The method of this application includes the following steps that:

$n$ types of compounds to be tested are composed into $m$ mixture systems, each of the mixture systems includes at least two of the $n$ types of compounds to be tested, and the types of the compounds to be tested are different in different mixture systems, a difference value in type of the compounds to be tested included in different mixture systems is within a first preset range, a same compound to be tested is included in at least two different mixture systems, a difference value in number of the mixture systems for each compound to be tested is within a second preset range, and a difference value in number of the compounds to be tested for each mixture system is within a third preset range;

$m$ portions of target solutions are prepared according to the $m$ mixture systems, and each portion of the target solution includes all of the compounds to be tested included in the mixture system and a target protein;

a response value of each mixture system's ability to interact with the target protein in each portion of the target solution; and, an interaction between any of the compounds to be tested included in each mixture system and the target protein is determined based on the response value of each mixture system' ability to interact with the target protein.

Further, the step of determining the interaction between any of the compounds to be tested included in each mixture system and the target protein according to the response value of each mixture system's ability to interact with the target protein includes steps of:

determining, according to the response value of each mixture system's ability to interact with the target protein, a contribution of each compound to be tested in each mixture system to the response value of the mixture system;

determining, if the contribution of a first compound to be tested in any of the mixture systems to the response value of any of the mixture systems is greater than or equal to a preset threshold, that the first compound to be tested has an interaction with the target protein, where the first compound to be tested is one of all the compounds to be tested included in any of the mixture systems.

Further, the step of composing the $n$ types of compounds to be tested into the $m$ mixture systems includes that: composing the $n$ types of compounds to be tested into the $m$ mixture systems according to an $m \times n$ permutation matrix S, each row in the permutation matrix S represents one mixture system, and each column represents one type of compound to be tested, the permutation matrix $S$ includes $m \times n$ indicators, the indicators are used to indicate whether the mixture system includes the compound to be tested corresponding to the column where the indicator is located, and the same compound to be tested is included in at least two of the mixture systems.

Further, the permutation matrix is obtained by the following steps:

mixing the $n$ types of compounds to be tested into $m$ mixture systems, and each compound to be tested is included in a different mixture systems, where $m \geq 3$, $n \geq 4$, $a \geq 2$, and $m$, $n$, and $a$ are all integers; the $m$ mixture systems are recorded as an $m \times n$ initial permutation matrix A of the compounds to be tested, and numerical values of various elements in the initial permutation matrix A of the compounds to be tested are all random number between 0 and 1;

performing a binary conversion on the initial permutation matrix A of the compounds to be tested, to find $a$ numerical values in each column of the matrix: $X_1$, $X_2$, $X_i$, ... $X_a$, and any numerical value $X_i$ in the $a$ numerical values is greater than other numerical values in this column, where $1 \leq i \leq a$, and $a$ is an integer; and to convert the $a$ numerical values in each column into a binary number of 1, and other numerical values into a binary number of 0, to obtain a conversion matrix $S$;

controlling the first preset range, the second preset range and the third preset range by performing an optimization of the initial permutation matrix A of the compounds to be tested, and the optimization includes a step of:

obtaining a value of an objective function L based on the following expression of the objective function:

$$L = Sum(S \cdot S^T - I) + Sum\big(RS - Mean(RS)\big)^2$$

where, RS is a sum of all rows of the conversion matrix $S$, $I$ is an identity matrix, and $S^T$ is a transposed matrix of $S$; the initial permutation matrix $A$ of the compound to be tested is optimized through an optimization algorithm to minimize the value of the objective function $L$; the binary conversion is performed on the initial permutation matrix $A$ of the compound to be tested when the value of the objective function $L$ is the minimum to obtain the permutation matrix S.

Further, the step of measuring the response value of each mixture system's ability to interact with the target protein in each portion of the target solution includes a step of:
measuring the response value of each mixture system's ability to interact with the target protein using a measurement method based on binding energy or activity of the interaction between the compound to be tested and the target protein.

Further, the target protein comes from a purified protein or cell lysate containing the target protein, and the response value of each mixture system's ability to interact with the target protein is quantitatively measured by either ABPP or PAL or TPP or LiP-MS.

Further, the step of determining the contribution of each compound to be tested in each mixture system to the response value of the mixture system according to the response value of each mixture system's ability to interact with the target protein includes steps of:

determining a response vector corresponding to each mixture system according to the response value of each mixture system's ability to interact with the target protein; and

normalizing each response vector to enable the numerical value of each response vector to be between 0 and 1; and the response vector $Y_i$ and the permutation matrix S meet a relational expression of: $Y_i = S \times \beta_j + R$,

where, $Y_i$ represents a response value of the ability of the mixture system identified as $i$ among the $m$ mixture systems to interact with the target protein, $\beta_j$ is a contribution of the compound to be tested identified as j in the mixture system identified as $i$ to the response value of the mixture system identified as $i$, and R is a residual vector with a length n; and

building, using a traditional statistical method or a machine learning method, a regression model, to optimally solve a numerical value of $\beta_j$ and to minimize the residual R.

Further, the first preset range, the second preset range and the third preset range are controlled by optimizing the initial permutation matrix A of the compounds to be tested, and the optimization steps include:

obtaining a value of an objective function L based on the following expression of the objective function:

$$L = Sum(S \cdot S^T - I) + Sum\big(RS - Mean(RS)\big)^2$$

where, RS is a sum of all rows of the conversion matrix S, $I$ is an identity matrix; the initial permutation matrix A of the compound to be tested is optimized through an optimization algorithm to minimize the value of the objective function L; a conversion is performed on the initial permutation matrix A of the compound to be tested when the value of the objective function L is the minimum to obtain an optimized permutation matrix S.

Further, the optimization algorithm includes a genetic algorithm and an ant colony algorithm.

Further, the traditional statistical method includes a least squares method, a LASSO regression method; and/or

The machine learning method includes a support vector machine and a random forest.

Compared with the existing technologies, the present application has the following technical effects:

In the method of improving the throughput of compound-protein interaction experiments provided by this application, multiple compounds to be tested are composed into multiple mixture systems according to a certain mixing rule, and corresponding relationships between abilities of the compounds to be tested to interact with the protein target and the mixture systems are established, and then a high-throughput analysis of the target protein corresponding to the compound to be tested is enabled. The method of the present application can increase a detection throughput of the existing compound to be tested-target protein experiments by more than 10 times, while saving more than 90% of the experimental cost and time, significantly reducing the cost of manpower, time and experimental consumables, which has significant economic significance.

## DESCRIPTION OF THE DRAWINGS

In order to illustrate the technical solutions of specific embodiments of the present application or the existing technologies more clearly, the drawings required to be used in the specific embodiments or the existing technologies will be briefly introduced below. It would be obvious that the following drawings are merely some implementations of the present application, and for persons of ordinary skill in the art, other drawings may also be obtained based on these drawings without exerting creative efforts.

FIG. 1 is an optimization flow chart for an initial permutation matrix A provided by an embodiment of the present application;

FIG. 2 is a schematic diagram of a permutation matrix S in an intermediate state during an optimization process provided in Example 1 of the present application;

FIG. 3 is a schematic diagram of an optimized permutation matrix S in a final state in the optimization process provided in Example 1 of the present application;

FIG. 4 is a schematic diagram showing that an objective function value L provided in Example 1 of the present application decreases to a constant value as the number of iterations increases;

FIG. 5 is a schematic diagram of the optimized permutation matrix S in the final state provided in Example 2 of the present application; and

FIG. 6 is a schematic diagram showing that the objective function value L provided in Example 2 of the present application decreases to a constant value as the number of iterations increases.

## DETAILED DESCRIPTION OF THE EMBODIMENTS

In order to make the technical problems, technical solutions and beneficial effects to be solved by this application much clearer, the present application will be further described in detail below in conjunction with the embodiments. It should be understood that the specific embodiments described here are only used to explain the present application and are not intended to limit the present application.

An embodiment of the present application provides a method for improving a throughput of compound-protein interaction experiments. The method of the present application includes the following steps (1), (2) and (3):

In step (1), $n$ types of compounds to be tested are composed into $m$ mixture systems, each mixture system includes at least two of the $n$ types of compounds to be tested, the compounds to be tested included in different mixture systems have different types, and a difference value in type of compounds to be tested included in different mixture systems is within a first preset range. The same compound to be tested is included in at least two different mixture systems, a difference value in number of mixture systems for each compound to be tested to be included within a second preset range, and a difference value in number of compounds to be tested contained in each mixture system is within a third preset range.

In step (2), *m* portions of target solutions are prepared according to the *m* mixture systems. Each portion of the target solution contains all the compounds to be tested included in the mixture system and a target protein.

In step (3), a response value of each mixture system's ability to interact with the target protein in each portion of the target solution is measured; an interaction between the target protein and any of the compounds to be tested included in each mixture system is determined based on the response value of each mixture system's ability to interact with the target protein.

The method of improving the throughput of compound-protein interaction experiments in this application uses multiple compounds to be tested to form multiple mixture systems according to certain mixing rules, and a correspondence relation between an ability of each compound to be tested to interact with the target protein and the mixture system is established. Then, a high-throughput analysis of the target protein corresponding to the compound to be tested is enabled. The method of this application can increase the detection throughput in the existing experiments on interaction between the compound to be tested and the target protein by more than 10 times, while saving more than 90% of the experimental cost and time, significantly reducing the cost of manpower, time and experimental consumables, which has significant economic significance.

In the present application, the "first preset range" in the above step (1) is controlled by controlling the types of compounds included in each mixture system to be as different as possible; the "second preset range" is controlled by controlling the number of the compounds to be tested included in each mixture system to be as equal as possible; the "third preset range" is controlled by controlling the number of the mixture systems of each compound to be tested to be as equal as possible.

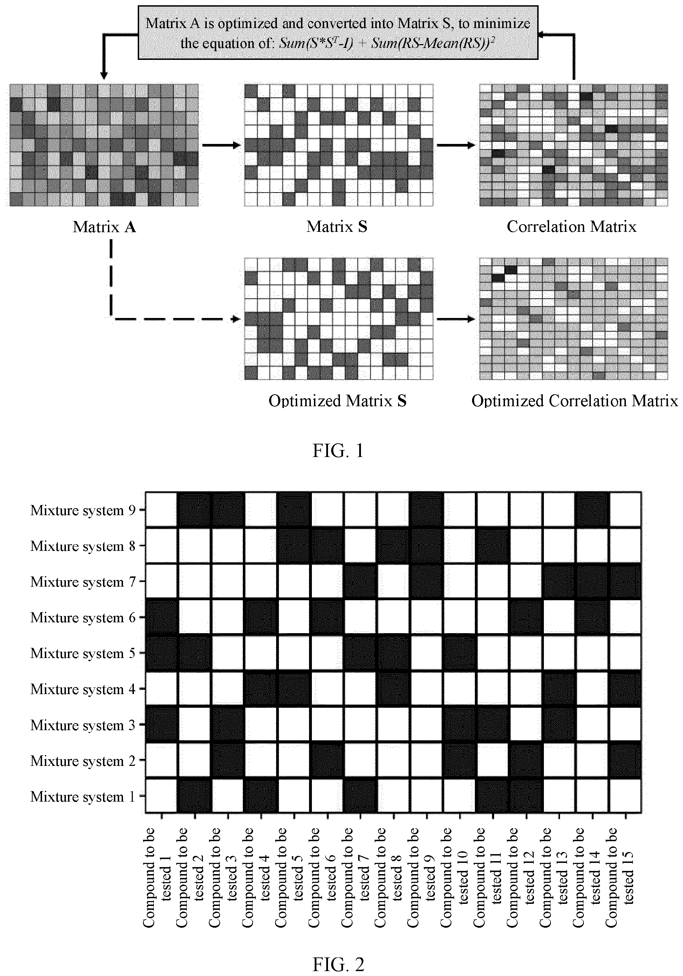

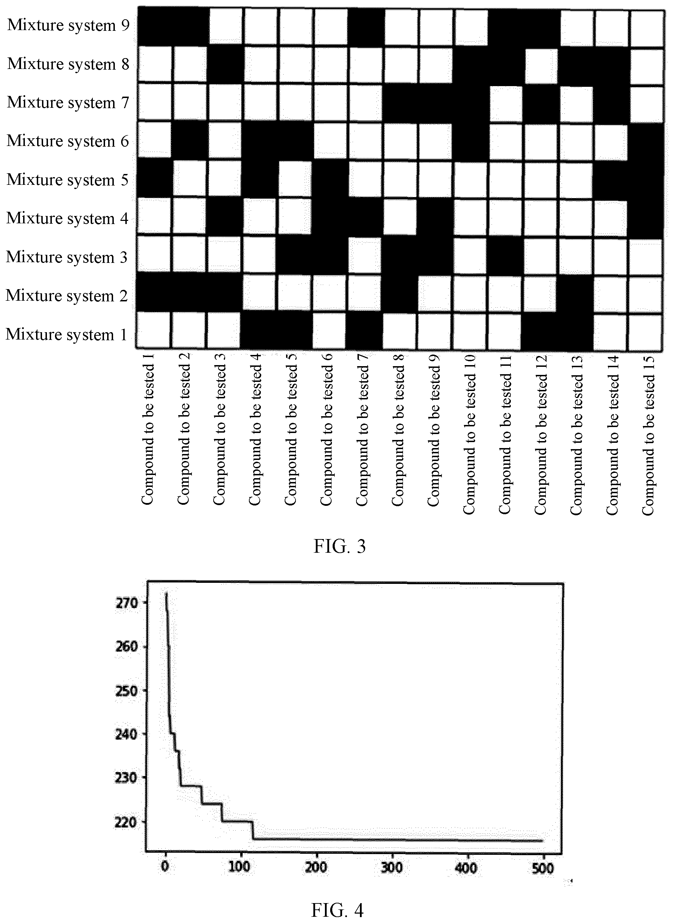

Further, in the above step (1), that "n types of compounds to be tested are composed into *m* mixture systems" particularly includes a step of: forming *n* types of compounds to be tested into *m* mixture systems according to an $m \times n$ optimized permutation matrix S. Each row in the permutation matrix S represents a mixture system, and each column represents a compound to be tested. The permutation matrix S includes $m \times n$ indicators. Each indicator is used to indicate whether the mixture system contains the compound to be tested corresponding to the column where the indicator is located. The same compound to be tested is present in at least two mixture systems. For example, in Example 1 of the present application, in the $9 \times 15$ permutation matrix S, the permutation matrix S indicates that 15 compounds to be tested are composed of 9 mixture systems. In FIG. 2 and FIG. 3, the ordinates 1 to 9 respectively represent 9 mixture systems formed by mixing, and the abscissas 1 to 15 respectively represent 15 compounds to be tested. In addition, as shown in FIG. 2 and FIG. 3, a black grid represents that the mixture system represented by this row contains the compound to be tested corresponding to this column, while a white grid represents that the mixture system represented by this row does not contain the compound to be tested corresponding to this column.

For example, in FIG. 3, the mixture system 1 represents that the mixture system contains the compound to be tested 4, the compound to be tested 5, the compound to be tested 7, the compound to be tested 12 and the compound to be tested 13.

Further, the above permutation matrix S may be obtained by the following method:

In this method, *n* types of compounds to be tested are mixed into *m* mixture systems, and each type of compound to be tested may be included in *a* different mixture systems, where $m \geq 3$, $n \geq 4$, $a \geq 2$, and *m, n,* and *a* are all integers. The *m* mixture systems are recorded as $m \times n$ initial permutation matrix A of the compounds to be tested. Numerical values of various elements in the initial permutation matrix A of the compounds to be tested are all random number between 0 and 1.

Binary conversion is performed on the initial permutation matrix A of the compounds to be tested: to find *a* numerical values $X_1, X_2, X_i, ... X_a$, in each column of the matrix, where any numerical value $X_i$ of the *a* numerical values is greater than other numerical values in the same column, where $1 \leq i \leq a$, and *a* is an integer. The numerical values in each column are converted into a binary number of 1, and the other numerical values in this column are converted into a binary number of 0, and then a conversion matrix S is obtained. Then the numbers 0 and 1 contained in the conversion matrix S are the indicators as above-mentioned. In the conversion matrix S, the numerical value 1 represents that the corresponding mixture system contains the compound to be tested, and the numerical value 0 represents that the corresponding mixture system does not contain the compound to be tested.

The above-mentioned "first preset range", "second preset range" and "third preset range" may be controlled by optimizing the initial permutation matrix A of the compounds to be tested. Specific optimization may be carried out through the following steps:

First, obtaining a value of an objective function L based on the objective function expressed as follows:

$$L = Sum(S \cdot S^T - I) + Sum\big(RS - Mean(RS)\big)^2$$

Where, RS is the sum of all rows of the permutation matrix S, and *I* is an identity matrix, the correlation between columns in the permutation matrix S may be obtained by $(S \cdot S^T - I)$ which is recorded as a correlation matrix. The first term $Sum(S \cdot S^T - I)$ in this objective function *L* is used to ensure that the correlation between columns in the matrix is minimal, and the second term $Sum(RS - Mean(RS))^2$ is used to ensure that the number of the compounds to be tested included in the mixture systems are close to each other.

Then, the initial permutation matrix *A* of the compounds to be tested may be optimized through an optimization algorithm to minimize the value of the objective function *L*. The optimization matrix *A* is subjected to the above binary conversion when the value of the objective function L is the minimum to obtain the optimized permutation matrix. Among them, the optimization algorithm includes but is not limited to a genetic algorithm, an ant colony algorithm, etc.

An optimization process of the initial permutation matrix A in this embodiment of the present application is shown in FIG. 1.

In this way, the present application maps *n* types of compounds to be tested composed of *m* mixture systems into an $m \times n$ permutation matrix, and then establishes a corresponding relationship between the ability of the compounds to be tested to interact with the target protein and the permutation matrix, thereby achieving a high-throughput analysis of the target protein corresponding to the compound to be tested located at a specific arrangement position in the permutation matrix.

In addition, in the above step (3), that "the interaction between the target protein and any of the compounds to be tested included in each mixture system is determined based on the response value of the ability of each mixture system to interact with the target protein" may particularly be determined through the following steps:

Determining, according to the response value of the ability of each mixture system to interact with the target protein, a contribution of each compound to be tested in each mixture system to the response value of the mixture system.

In case that the contribution of a first compound to be tested in any mixture system to the response value of any mixture system is greater than or equal to a preset threshold, then it is determined that the first compound to be tested interacts with the target protein, where the first compound to be tested is one of all compounds to be tested included in any mixture system.

Further, the step of determining the contribution of each compound to be tested in each mixture system to the response value of the mixture system includes that:

A response vector corresponding to each mixture system is determined according to the response value of each mixture system' ability to interact with the target protein. Particularly, any measurement method for the interaction between the compound to be tested and the target protein based on binding energy or activity in the existing technologies may be used to measure the response value of each mixture system's ability to interact with the target protein. For example, the response value of each mixture system's ability to interact with the target protein may be quantitatively measured by a method based on either ABPP (activity-based protein profiling) or PAL (photoaffinity) or TPP (thermal proteomics) or LiP-MS (limited proteolysis mass spectrometry). In specific Examples 1 and 2 of this application, a single temperature point TPP method was used to measure the interaction between each mixed system and the target protein.

Each response vector is normalized so that the numerical value of the normalized response vector is between 0 and 1; the response vector $Y_i$ and the permutation matrix S meet a relational expression of:

$$Y_i = S \times \beta_j + R$$

Where, $Y_i$ represents a response value of the ability of a mixture system identified as *i* among the m mixture systems to interact with the target protein, and $\beta_j$ is a contribution of a compound to be tested identified as *j* in the mixture system identified as *i* to the response value of the mixture system identified as *i*, and R is a residual vector with a length n.

A regression model is built by using a traditional statistical method or a machine learning method, to optimize a solution of the numerical value of $\beta_j$, and to minimize the residual *R*. The traditional statistical method includes but is not limited to a least square method, a LASSO regression method, etc., and the machine learning method includes but is not limited to a support vector machine, a random forest regression, etc.

In specific embodiments of this application, the LASSO regression may be used to solve an optimal solution of $\beta$ as follows:

$$\beta = \arg\min_{\beta} \left( \frac{1}{n} \left\| Y - S \square \beta \right\|_2^2 + \lambda \left\| \beta \right\|_1 \right)$$

Where, $\lambda$ is a penalty term, which is used to adjust a compression degree of $\beta$. In the specific embodiments 1 and 2 of the present application, the value of $\lambda$ is 0.1, and the threshold is 0.1. If the calculated value of $\beta_i$ corresponding to a

certain compound to be tested is higher than 0.1, then it may be determined that the corresponding compound to be tested has an interaction with the target protein.

The method for analyzing an interaction between a compound and a protein in the embodiments of the present application are illustrated through the following specific examples.

**Example 1**

Example 1 provides a method for improving a throughput of compound-protein interaction experiments, which includes the following steps S01, S02, S03, S04 and S05.

In step S01: Fifteen drugs to be tested (i.e., the compounds to be tested) were given: Palbociclib, Panobinostat, Raltitrexed, Methotrexate, Vemurafenib, Fimepinostat, SCIO-469, SL-327, 5-Fluorouracil, Olaparib, Belumosudil, OTS964, Parthenolide, CCT137690, Belumosudil, which are sequentially numbered as compound to be tested 1, compound to be tested 2, compound to be tested 3, ... and compound to be tested 15. It is assumed here that the above fifteen compounds to be tested are mixed into nine mixture systems: mixture system 1, mixture system 2, mixture system 3, ... and mixture system 9, and each compound to be tested was included in three different mixture systems, that is, $m = 9$, $n = 15$, $a = 3$. The nine mixture systems are mapped into a $9 \times 15$ initial permutation matrix A of the compounds to be tested, and the initial permutation matrix A is randomly initialized so that the numerical values in the matrix $A$ are random floating-point numbers between 0 and 1.

In step S02: A binary conversion was performed on the initial permutation matrix $A$ of the compounds to be tested to find three numerical values: $X_1$, $X_2$, $X_3$, in each column. Any one of the three numerical values is greater than the other six numerical values in the column. The three numerical values in each column were converted into a binary number of 1, and the other values in this column were converted into a binary number of 0, and then a conversion matrix was obtained;

In step S03: A value of an objective function L was obtained through the following expression of the objective function:

$$L = Sum(S \cdot S^T - I) + Sum\big(RS - Mean(RS)\big)^2$$

Where, $RS$ is the sum of all rows of the above conversion matrix $S$, $I$ is the identity matrix, and $S^T$ is a transposed matrix of S.

The initial permutation matrix A of the compounds to be tested was iteratively optimized through a genetic algorithm to minimize the value of the objective function $L$; the permutation matrix $A$ is subjected to the binary conversion in step S02 when the value of the objective function $L$ is the minimum to obtain the optimized permutation matrix $S$ (as shown in FIG. 3), the optimized permutation matrix $S$ is the final permutation matrix. FIG. 2 shows a schematic diagram of the permutation matrix of an intermediate state during the optimization process. In FIGS. 2 and 3, the black grid represents the numerical value 1, and the white grid represents the numerical value 0. During the iteration process, the value of the objective function $L$ decreases to a constant value as the number of iterations increases, as shown in FIG. 4. The fifteen compounds to be tested were mixed according to the finally-obtained $9 \times 15$ optimized permutation matrix shown in FIG. 3, and nine mixture systems were obtained.

In step S04: DMSO as a solvent was added to each mixture system in step S03 to enable a concentration of each drug to reach 40 $\mu$M, and then the single temperature point TPP method is applied to measure the interaction between each mixture system and the protein. The specific experimental steps are that: equal amounts of K562 cell lysate were mixed with a drug mixture system, incubated at room temperature for 10 minutes, then heated at 52°C for 3 minutes, and then quickly cooled to 4°C on a PCR machine. The samples were centrifuged at 21,000 rcf for 20 minutes at 4 °C, and the supernatant was collected. According to the mass spectrometry-based proteomics quantification method, each sample was enzymatically digested and TMT labeled, and then the LC-MS mass spectrometry was used for measurement to obtain the content of protein. Based on the principle of the TPP method, the thermal stability of the protein can be improved through a combination with compounds. Therefore, if a protein can interact with the mixed system, then the content of protein measured by mass spectrometry will be higher, and vice versa.

In step S05: Nine measured values $Y_i$ obtained from the interaction between the nine mixture systems and the target protein are marked as response vectors Y; the response vectors $Y$ were normalized so that the numerical values of the response vectors are between 0 and 1. The response vector $Y$ and the permutation matrix S meet a relational expression of:

$$Y_i = S \times \beta_j + R$$

Where, $Y_i$ represents a response value of the ability of the mixture system identified as $i$ among the nine mixture systems to interact with the target protein, and $\beta_j$ is a contribution of the compound to be tested identified as j in the mixture system identified as *i* to the response value of the mixture system identified as *i*, $\beta_j$ is a coefficient vector with a length n, and R is a residual vector with a length n.

the LASSO regression method is applied to obtain an optimal solution of β based on the following expression:

$$\beta = \arg\min_{\beta}\left(\frac{1}{n}\left\|Y - S\square\beta\right\|_2^2 + \lambda\left\|\beta\right\|_1\right)$$

Where, λ is a penalty term, which is used to adjust a compression degree of *β*, in this embodiment, the value of λ is 0.1 and the threshold is 0.1. If the calculated value of $\beta_i$ corresponding to a certain drug to be tested is higher than 0.1, then it may be determined that the corresponding drug compound to be tested has an interaction with the target protein.

Finally, the targets of the fifteen compounds to be tested were identified by preparing nine mixture systems, eleven of the fifteen targets were successfully identified (see Table 1 below), with a success rate of 73.3%. On average, only 0.6 samples need to be prepared for each compound to be tested.

For comparison, in the conventional single-temperature TPP method (Ball et al, Commun. Biol. 2020(3). 75), it is necessary to set up four administration group samples and four control group sample for a target identification of each drug compound to be tested, that is, a target identification of one drug requires preparation of eight samples. Therefore, the method provided by this example in the present application increases the detection throughput by 8 / 0.6 = 13.3 times compared to the conventional method, and reduces the experimental cost by (8 - 0.6) / 8 = 92.5%.

Table 1. Target identification results of 15 drug compounds to be tested

| Drug Name | Drug Target(s) | Successfully Identified Target(s) |
|---|---|---|
| Palbociclib | CDK4, CDK6 | CDK4, CDK6 |
| Panobinostat | HDAC family | HDAC1, HDAC2, HDAC10 |
| Raltitrexed | TYMS | TYMS |
| Methotrexate | DHFR | DHFR |
| Vemurafenib | BRAF | BRAF |
| Fimepinostat | HDAC1 | HDAC1 |
| SCIO-469 | MAPK family | MAPK 14 |
| SL-327 | MEK family | MAP2K1, MAP2K2 |
| 5-Fluorouracil | TYMS | - |
| Olaparib | PARP1 | PARP1 |
| Belumosudil | ROCK family | - |
| OTS964 | MAPK 14 | - |
| Parthenolide | HDAC family | - |
| CCT137690 | AURK family | AURKA |
| Ralimetinib | MAPK family | MAPK 14 |

The method provided by Example 1 of the present application is an effective method to improve the throughput of identification of pharmaceutical compound targets. First, an optimized permutation matrix *S* is constructed through an optimization algorithm, and a variety of compounds to be tested are composed of a mixture system according to the optimized permutation matrix S. Then, the existing method for measurement of a compound-target interaction is combined, and finally a statistical method is applied to analyze a corresponding relationship of compound-target interaction, which can increase the number of compounds that can be analyzed by more than 10 times at the same experimental cost, and thus can greatly reduce the cost of manpower, time and experimental consumables and possess significant economic benefits.

**Example 2**

Example 2 provides a method for improving a throughput of compound-protein interaction experiments, which includes the following steps that:

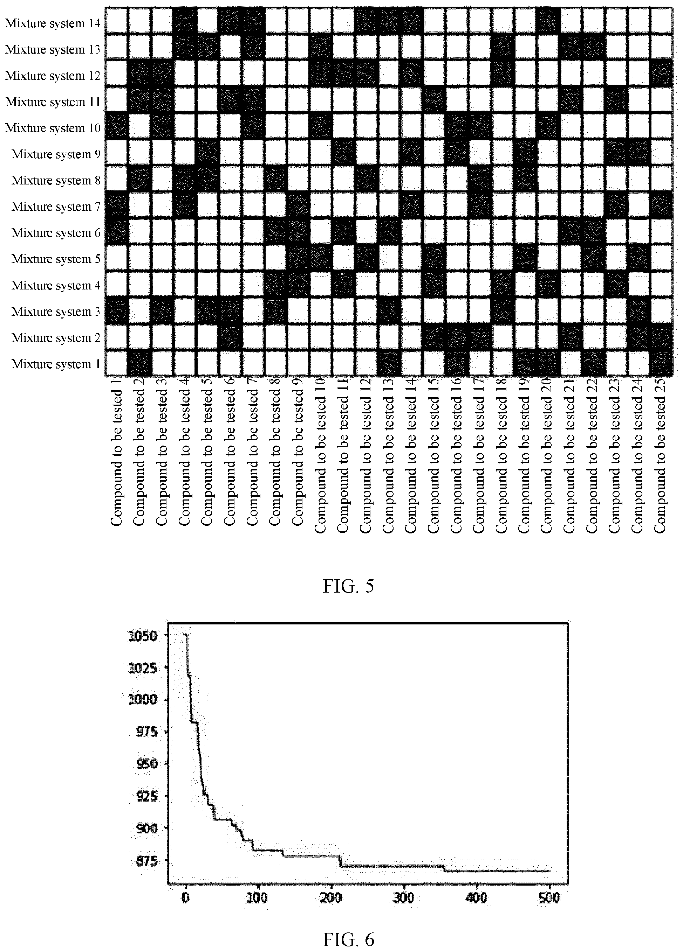

In addition to the fifteen compounds to be tested given in Example 1, the following compounds of Tioxolone, Parthenolide, Abemaciclib, Caffeic acid phenethyl ester, RG2833, Encorafenib, TAK-285, CNX-774, Dienogest, ZM241385 were added, that is, a total of twenty-five compounds to be tested were given, which are sequentially numbered as compound to be tested 1, compound to be tested 2, compound to be tested 3, ... and compound to be tested 25. It is assumed that the above twenty-five compounds to be tested are mixed into fourteen mixture systems: mixture system 1, mixture system 2, mixture system 3, ... and mixture system 14. Each compound to be tested was included in four different mixture systems. That is, m=14, n=25, a=4. In accordance with the steps in Example 1, the genetic algorithm is applied to optimize the permutation matrix $S$, and the optimized permutation matrix S is shown in FIG. 5. In FIG. 5, the black grid represents the numerical value of 1, and the white grid represents the numerical value of 0. During the iteration process, the value of the objective function $L$ decreases to a constant value as the number of iterations increases, as shown in FIG. 6. The twenty-five compounds to be tested were mixed according to the finally obtained 14×25 optimized permutation matrix S shown in FIG. 5, to obtain fourteen mixture systems. The remaining operation steps are consistent with Example 1.

Finally, the targets of twenty-five compounds to be tested were identified by preparing fourteen mixture systems, fourteen of the twenty-five targets were successfully identified (see Table 2 below), with an identification success rate of 56%. On average, only 0.56 samples need to be prepared for each compound to be tested.

For comparison, in the conventional single-temperature TPP method (Ball et al, Commun. Biol. 2020(3). 75), it is necessary to set up four administration group samples and four control group sample for a target identification of each drug compound to be tested, that is, a target identification of one drug requires preparation of eight samples. Therefore, the method provided by this example in the present application increases the detection throughput by 8 / 0.56 = 14.3 times compared with the conventional method, and reduces the experimental cost by (8 - 0.56) / 8 = 93.0%.

Table 2. Target identification results of twenty-five drug compounds to be tested

| Drug Name | Drug Target(s) | Successfully Identified Target(s) |
|---|---|---|
| Palbociclib | CDK4, CDK6 | CDK4, CDK6 |
| Panobinostat | HDAC family | HDAC1, HDAC2 |
| Raltitrexed | TYMS | TYMS |
| Methotrexate | DHFR | DHFR |
| Vemurafenib | BRAF | BRAF |
| Fimepinostat | HDAC1 | HDAC1 |
| SCIO-469 | MAPK family | MAPK 14 |
| SL-327 | MEK family | MAP2K1, MAP2K2 |
| 5-Fluorouracil | TYMS | - |
| Olaparib | PARP1 | PARP1 |
| Belumosudil | ROCK family | - |
| OTS964 | MAPK 14 | - |
| Parthenolide | HDAC family | - |
| CCT137690 | AURK family | - |
| Ralimetinib | MAPK family | MAPK 14 |
| Tioxolone | CA13 | - |
| Parthenolide | NFKB | NFKB |
| Abemaciclib | CDK4, CDK6 | CDK4 |
| Caffeic acid phenethyl ester | NFKB | - |
| RG2833 | HDAC1 | HDAC1 |
| Encorafenib | BRAF | BRAF |
| TAK-285 | EGFR | - |

(continued)

| Drug Name | Drug Target(s) | Successfully Identified Target(s) |
|---|---|---|
| CNX-774 | BTK | - |
| Dienogest | ATP1A | - |
| ZM241385 | ADORA1 | - |

The above examples merely express several implementations of the present application, and the descriptions are relatively specific and detailed, which should not be construed as limiting the protection scope of the present application. It should be noted that, for those of ordinary skill in the art, several modifications and improvements may be made without deviating from the concept of the present application, and these modifications and improvements shall all fall within the protection scope of the present application. Therefore, the protection scope of this patent application should be determined by the appended claims.

**Claims**

1. A method for improving a throughput of compound-protein interaction experiments, **characterized by** comprising:

    composing $n$ types of compounds to be tested into $m$ mixture systems, each of the mixture systems comprising at least two of the n types of compounds to be tested, wherein the compounds to be tested comprised in different mixture systems have different types, and a difference value in type of the compounds to be tested comprised in different mixture systems is within a first preset range, a same compound to be tested is comprised in at least two different mixture systems, a difference value in number of the mixture systems for each compound to be tested is within a second preset range, and a difference value in number of the compounds to be tested comprised in each mixture system is within a third preset range;
    preparing $m$ portions of target solutions according to the $m$ mixture systems, wherein each portion of the target solution contains all of the compounds to be tested comprised in the mixture system and a target protein;
    measuring, in each portion of the target solution, a response value of each mixture system' ability to interact with the target protein; and
    determining, according to the response value of each mixture system' ability to interact with the target protein, an interaction between any of the compounds to be tested comprised in each mixture system and the target protein.

2. The method according to claim 1, **characterized in that**, said determining, according to the response value of each mixture system's ability to interact with the target protein, the interaction between any of the compounds to be tested comprised in the mixture system and the target protein, comprises:

    determining, according to the response value of each mixture system's ability to interact with the target protein, a contribution of each compound to be tested in each mixture system to the response value of the mixture system; and
    determining, when the contribution of a first compound to be tested in any of the mixture systems to the response value of any of the mixture systems is greater than or equal to a preset threshold, that the first compound to be tested has an interaction with the target protein, wherein the first compound to be tested is one of all the compounds to be tested comprised in any of the mixture systems.

3. The method according to claim 1, **characterized in that**, said composing the $n$ types of compounds to be tested into the $m$ mixture systems comprises:
    composing the $n$ types of compounds to be tested into the $m$ mixture systems according to an $m \times n$ permutation matrix S, each row in the permutation matrix S represents one of the mixture systems, and each column represents one type of the compounds to be tested, the permutation matrix S comprises $m \times n$ indicators, the indicators are used to indicate whether the mixture system comprises the compound to be tested corresponding to the column where the indicator is located, and the same compound to be tested is comprised in at least two of the mixture systems.

4. The method according to claim 3, **characterized in that**, the permutation matrix S is obtained by:

    mixing the $n$ types of compounds to be tested into $m$ mixture systems, and each compound to be tested is comprised in a different mixture systems, wherein $m \geq 3, n \geq 4, a \geq 2$, and $m, n,$ and $a$ are all integers; the $m$ mixture systems are recorded as an $m \times n$ initial permutation matrix $A$ of the compounds to be tested, and numerical values of various elements in the initial permutation matrix A of the compounds to be tested are all random number between 0 and 1;

performing a binary conversion on the initial permutation matrix A of the compounds to be tested, to find $a$ numerical values in each column of the matrix: $X_1, X_2, X_i, ... X_a$, any numerical value $X_i$ in the $a$ numerical values is greater than other numerical values in this column, wherein $1 \le i \le a$, and $a$ is an integer; and to convert the $a$ numerical values in each column into a binary number of 1, and other numerical values into a binary number of 0, to obtain a conversion matrix $S$; and

controlling the first preset range, the second preset range and the third preset range by performing an optimization of the initial permutation matrix A of the compounds to be tested, and the optimization comprises:

obtaining a value of an objective function L based on an expression of:

$$L = Sum(S \cdot S^T - I) + Sum\left(RS - Mean(RS)\right)^2$$

wherein, RS is a sum of all rows of the conversion matrix $S$, $I$ is an identity matrix, and $S^T$ is a transposed matrix of S; the initial permutation matrix $A$ of the compound to be tested is optimized through an optimization algorithm to minimize the value of the objective function $L$; the binary conversion is performed on the initial permutation matrix $A$ of the compound to be tested when the value of the objective function $L$ is the minimum to obtain the permutation matrix $S$.

5. The method according to claim 1, **characterized in that**, said measuring, in each portion of the target solution, the response value of each mixture system's ability to interact with the target protein comprises:

measuring, using a measurement method based on binding energy or activity of the interaction between the compound to be tested and the target protein, the response value of each mixture system's ability to interact with the target protein.

6. The method according to any one of claims 1 to 5, **characterized in that**, the target protein is derived from a purified protein or celllysate containing the target protein, and the response value of each mixture system's ability to interact with the target protein is quantitatively measured by either ABPP or PAL or TPP or LiP-MS.

7. The method according to claim 3 or 4, **characterized in that**, said determining, according to the response value of each mixture system's ability to interact with the target protein, the contribution of each compound to be tested in each mixture system to the response value of the mixture system, comprises:

determining a response vector corresponding to each mixture system according to the response value of each mixture system's ability to interact with the target protein;

normalizing each response vector to enable the numerical value of each response vector to be between 0 and 1; and the response vector $Y_i$ and the permutation matrix S meet a relational expression of: $Y_i = S \times \beta_j + R,$ wherein, $Y_i$ represents a response value of the ability of the mixture system identified as $i$ among the $m$ mixture systems to interact with the target protein, $\beta_j$ is a contribution of the compound to be tested identified as $j$ in the mixture system identified as $i$ to the response value of the mixture system identified as $i$, and $R$ is a residual vector with a length n; and

building, using a traditional statistical method or a machine learning method, a regression model, to optimally solve a numerical value of $\beta_j$ and to minimize the residual $R$.

8. The method according to claim 4, **characterized in that**, the optimization algorithm comprises a genetic algorithm and an ant colony algorithm.

9. The method according to claim 7, **characterized in that**, the traditional statistical method comprises a least squares method and a LASSO regression method.

10. The method according to claim 7, **characterized in that**, the machine learning method comprises a support vector machine and a random forest.

Matrix **A**        Matrix **S**        Correlation Matrix

Optimized Matrix **S**        Optimized Correlation Matrix

FIG. 1

FIG. 2

FIG. 3

FIG. 4

FIG. 5

FIG. 6

| INTERNATIONAL SEARCH REPORT | International application No. |
|---|---|
| | **PCT/CN2023/098376** |

**A. CLASSIFICATION OF SUBJECT MATTER**

G01N33/68(2006.01)i；G16B 40/00(2019.01)i

According to International Patent Classification (IPC) or to both national classification and IPC

**B. FIELDS SEARCHED**

Minimum documentation searched (classification system followed by classification symbols)

IPC：G01N G16B

Documentation searched other than minimum documentation to the extent that such documents are included in the fields searched

Electronic data base consulted during the international search (name of data base and, where practicable, search terms used)

CNTXT; ENTXTC; WPABSC; VEN; ENTXT; CNKI; 百度学术, BAIDU SCHOLAR; IEEE; SpringerLink; Elsevier Science: 南方科技大学, 纪宏超, 陈顺兴, 压缩感知, 压缩感测, 靶点, 目标函数, 遗传算法, 残差, 损失函数, 矩阵, 池, 通量, CS, LASSO, Pool+, Matrix, Throughput, Target, Compressed Sensing, cost function, objective function

**C. DOCUMENTS CONSIDERED TO BE RELEVANT**

| Category* | Citation of document, with indication, where appropriate, of the relevant passages | Relevant to claim No. |
|---|---|---|
| PX | JI, Hongchao et al. "Matrix-Augmented Pooling Strategy for High-Throughput Target Deconvolution Reveals Cell Type Specific Off-targets and Responses" *Cell Chemical Biology,* 21 November 2022 (2022-11-21), ISSN: 2451-9448, pages 2-27 | 1-10, |
| X | SABYASACHI GHOSH et al. "A Compressed Sensing Approach to Pooled RT-PCR Testing for COVID-19 Detection" *IEEE Open Journal of Signal Processing,* Vol. 2, 27 April 2021 (2021-04-27), ISSN: 2644-1322, abstract, and text, page 249, left-hand column, paragraph 2-page 263, right-hand column, paragraph 1 | 1-3, 5-7, 9, 10 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 August 2023** | **08 September 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2023/098376** |

### C. DOCUMENTS CONSIDERED TO BE RELEVANT

| Category* | Citation of document, with indication, where appropriate, of the relevant passages | Relevant to claim No. |
|---|---|---|
| X | Ramy Mourad et al. "Designing Pooling Systems for Noisy High-Throughput Protein-Protein Interaction Experiments Using Boolean Compressed Sensing" *IEEE/ACM TRANSACTIONS ON COMPUTATIONAL BIOLOGY AND BIOINFORMATICS*, Vol. 10, No. 6, 24 October 2013 (2013-10-24), ISSN: 1557-9964,<br>abstract, and text, page 1479, right-hand column, last paragraph-page 1488, right-hand column, last paragraph | 1-3, 5-7, 9, 10 |
| A | CN 103116713 A (ZHEJIANG UNIVERSITY) 22 May 2013 (2013-05-22)<br>entire document | 1-10 |
| A | CN 104215550 A (NANJING MEDICAL UNIVERSITY) 17 December 2014 (2014-12-17)<br>entire document | 1-10 |
| A | JP 2007143422 A (TOYO BOSEKI KABUSHIKI KAISHA) 14 June 2007 (2007-06-14)<br>entire document | 1-10 |

Form PCT/ISA/210 (second sheet) (July 2022)

**INTERNATIONAL SEARCH REPORT**
**Information on patent family members**

International application No.

**PCT/CN2023/098376**

| Patent document cited in search report | | | Publication date (day/month/year) | Patent family member(s) | | | Publication date (day/month/year) |
|---|---|---|---|---|---|---|---|
| CN | 103116713 | A | 22 May 2013 | CN | 103116713 | B | 16 September 2015 |
| CN | 104215550 | A | 17 December 2014 | None | | | |
| JP | 2007143422 | A | 14 June 2007 | None | | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210638301

**Non-patent literature cited in the description**

- **BALL et al.** *Commun. Biol*, 2020 (3), 75
- **BALL et al.** *Commun. Biol.*, 2020 (3), 75